**Europäisches Patentamt**

**European Patent Office**

**Office européen des brevets**

(11) Veröffentlichungsnummer: **0 232 216 B1**

(12) **EUROPÄISCHE PATENTSCHRIFT**

(45) Veröffentlichungstag der Patentschrift:
**08.01.92 Patentblatt 92/02**

(51) Int. Cl.⁵: **G01N 33/68, G01N 33/52**

(21) Anmeldenummer: **87730009.5**

(22) Anmeldetag: **29.01.87**

(54) **Schwangerschaftsnachweis mit "early pregnancy factor".**

(30) Priorität: **30.01.86 DE 3603053**

(43) Veröffentlichungstag der Anmeldung:
**12.08.87 Patentblatt 87/33**

(45) Bekanntmachung des Hinweises auf die
Patenterteilung:
**08.01.92 Patentblatt 92/02**

(84) Benannte Vertragsstaaten:
**AT BE CH DE ES FR GB GR IT LI LU NL SE**

(56) Entgegenhaltungen:
**FR-A- 2 544 080**
**US-A- 4 491 634**
**CHEMICAL ABSTRACTS, vol. 104, no. 15, 15**
**April 1986, Columbus, OH (US); R.COCCHIARA**
**et al., p. 97, no. 123390a**
**CHEMICAL ABSTRACTS, vol. 105, no. 15, 13**
**Oct 1986, Columbus, OH (US); H.R.TINNE-**
**BERG et al., p. 482, no. 131702q**

(73) Patentinhaber: **SCHERING**
**AKTIENGESELLSCHAFT Berlin und**
**Bergkamen**
**Müllerstrasse 170/178 Postfach 65 03 11**
**W-1000 Berlin 65 (DE)**

(72) Erfinder: **Maly, Friedrich E.,Dr.**
**Elisabethen Str. 43**
**CH-3014 Bern (CH)**
Erfinder: **de Weck, Alain L., Prof. Dr.**
**14 Grand Place**
**CH-1700 Fribourg (CH)**
Erfinder: **Henderson, David, Dr.**
**Jahnstr. 17**
**W-1000 Berlin 28 (DE)**

## Beschreibung

EPF (Early Pregnancy Factor) ist ein Protein, das während der Schwangerschaft im Blut oder Harn oder in biochemischen Derivaten von Blut oder Harn von Schwangeren auftritt. Außer in der Schwangerschaft kommt EPF nur bei Keimdrüsentumoren (Hoden- und Ovarientumoren) vor [Aust. J. Biol. Sci. 37 (1984) 393-107].

EPF bindet an Lymphocyten und kann im Rosetteninhibitionstest nachgewiesen werden. Der Test beruht darauf, daß EPF die Inhibierung der aktiven Rosettenbildung zwischen Lymphozyten und heterologen Erythrocyten durch Antilymphocytenserum verstärkt [Proc R. Soc. London Ser. B. 193 (1976) 113-119].

Im Rosetteninhibitionstest kann EPF im Serum gravider Frauen schon innerhalb von 21 Stunden nach der Konzeption (Verschmelzung von Ovum und Spermien) und 2 - 3 Tage später auch im Harn dieser Frauen nachgewiesen werden.

Bei der In-Vitro-Fertilisation wird ein erfolgreicher Embryo-Transfer durch das Auftreten von EPF ca. 3 Tage nach dem Transfer angezeigt (Ann. N.Y. Acad. Sci. 442:420-8, 1985).

Mit Hilfe von EPF läßt sich demnach eine Schwangerschaft zu einem sehr frühen Zeitpunkt (1 - 5 Tage nach der Konzeption) nachweisen. Der Rosotteninhibitionstest ist jedoch sehr arbeits- und zeitaufwendig; er ist außerdem mit einer hohen Fehlerquote behaftet. Eine Schwangerschaft wird oft erst nach wiederholten Tests entdeckt. Umgekehrt sind für den Ausschluß einer Schwangerschaft mehrere Wiederholungen des Tests erforderlich.

Es besteht daher ein großes Interesse an einem einfachen und zuvorlässigen Verfahren zum frühen Nachweis einer Schwangerschaft mit EPF.

Es wurde nun gefunden, daß EPF die durch eine Leukozytenpräparation oder bestimmten Zell-Linien hervorgerufene Bildung von elektronisch angeregten Zuständen oder Radikalen, insbesondere Sauerstoff-Radikalen und deren Folgeprodukte beeinflußt. Wenn man EPF-haltiges Serum bzw. EPF-haltiges Harnkonzentrat mit einer Leukozytenpräparation inkubiert, die Zellen wäscht und geeignet stimuliert, kann man die Bildung von elektronisch angeregten Zuständen oder Radikalen, insbesondere Sauerstoff-Radikalen oder deren Folgeprodukte messen. Im Falle von Sauerstoff-Radikalen, gemessen beispielsweise mit Lumineszenz, Cytochrom C-Reduktion oder $H_2O_2$-Bestimmung wird im Vergleich zu den nicht mit EPF behandelten Kontrollen eine Veränderung des Radikal-Fluxes gefunden. Bei den Kontrollen werden die Leukozytenpräparation oder Zell-Linien mit Nichtschwangeren-Serum oder -Harnkonzentrat oder deren biochemischen Derivaten inkubiert.

Die Erfindung betrifft demnach ein Verfahren zum frühen Nachweis einer Schwangerschaft durch Bestimmung von EPF (Early Pregnancy Factor) im Blut oder Harn oder in biochemischen Derivaten von Blut oder Harn von Schwangeren nach Ausschluß von Keimdrüsentumoren, dadurch gekennzeichnet, daß man die durch EPF beeinflußte Bildung von elektronisch angeregten Zuständen oder Radikalen, insbesondere Sauerstoff-Radikalen oder deren Folgeprodukte, einer Leukozytenpräparation oder menschlicher oder tierischer mononukleärer Zellen oder der "Holt"-B-Zelle-Linie, der HL-60-Zell-Linie oder von Makrophagen-Zell-Linien mißt.

Das erfindungsgemäße Verfahren ist auch geeignet zur Diagnose von Keimdrüsentumoren nach Ausschluß einer Schwangerschaft.

Ferner ist das erfindungsgemäße Verfahren geeignet zum Nachweis eines erfolgreichen Embryotransfers nach einer In-Vitro-Fertilisation oder zum Nachweis der Fertilisation nach einer künstlichen Insemination.

Das erfindungsgemäße Verfahren ist auch zur Anwendung im Veterinärbereich geeignet, vor allem bei landwirtschaftlichen Nutztieren, wie zum Beispiel bei Pferden, Rindern, Schweinen und Schafen. Bei der künstlichen Insemination ist die Erfolgsrate zum Beispiel enttäuschend niedrig, und es ist für den Züchter sehr wichtig, frühzeitig zu wissen, ob die Insemination erfolgreich war. Im folgenden werden die bevorzugte Ausführungsform (A) sowie alternative Auführungsformen (B) beschrieben.

## A Leukozytenpräparation

Blutentnahme von gesunden männlichen Spendern. Antikoagulation mit 1/10 Na Citrat 3,8 %. Gewinnung der mononukleären Leukozyten durch Zentrifugation über einen einstufigen Ficoll-Hypaque Gradienten (Dichte 1,077). Mehrmaliges Waschen der mononukleären Zellen mit Hank's Balanced Salt Solution ohne $Ca^{++}$, $Mg^{++}$ und ohne Phenolrot (HBSS). Aufnehmen der Zellen in Hepes-gepuffertem (pH 7.4) Minimal Essential Medium mit 100 µg/ml bovinem Serumalbumin (BSA-MEM) ohne Phenolrot und Einstellen auf eine Zelldichte von $20x10^6$/ml. In Luminometer-Röhrchen (3M, Lumacuvetten) werden je 50 µl dieser Suspension vorgelegt, entsprechend $1x10^6$ mononukleären Zellen/Röhrchen, und bis zur Messung bei +4 °C gelagert, maximal 8 Stunden.

Menschliche mononukleäre Zellen können auch von weiblichen Blutspendern gewonnen werden, wenn eine Schwangerschaft ausgeschlossen ist und Keimdrüsentumore nicht vorliegen.

**B Zell-Linien**

Alternativ können menschliche mononukleäre Zellen aus anderen quellen (z.B. Aveolar-Makrophagen, Peritonealzellen oder aus Knochenmark gewonnene Zellen) oder tierische mononukleäre Zellen (z.B. Maus-Peritonealzellen oder Maus-Knochenmarkskulturen) oder menschliche oder tierische Zell-Linien eingesetzt werden, die die Fähigkeit zur Bildung von elektronisch angeregten Zuständen oder Radikalen, insbesondere Sauerstoff-Radikalen oder deren Folgeprodukte besitzen (z.B. "Holt" -B-Zell-Linie, HL-60 und diverse Makrophagen-Linien).

Als EPF-Quellen werden verwandt:

Serum, Plasma, Urinkonzentrat oder biochemische Derivate dieser Materialien von Schwangeren. Als biochemische Derivate kommen durch Ultrafiltration, chromatographische oder elektrophoretische Trennung oder Lyophilisation gewonnene Extrakte, Fraktionen oder Konzentrate dieser Materialien infrage (J. Immunol. Methods 70 (1981) 1 - 11).

Als Kontrollen werden die entsprechenden Materialien der Nicht-Schwangeren vor dem Embryotransfer oder der Insemination eingesetzt.

Zu 1x10⁶ mononukleären Zellen in einem Luminometer-Röhrchen werden 100 µl Schwangeren- bzw. Kontroll-Serum pipettiert und 30 Minuten bei 37 °C inkubiert. Danach wird mehrere Male mit HBSS gewaschen. Schließlich werden die Zellen in 500 µl HBSS als Meßmedium aufgenommen.

Zellzahl, Probenmenge und Inkubationsdauer können verändert werden. Als Wasch-bzw. Messmedium kommen auch andere isotonische, etwa neutral gepufferte Lösungen, infrage. [Meth. Enzymology 32 (1974) 633 und 57 (1978) 462].

Zur Erfassung der EPF-Aktivität werden die mononukleären Zellen zur Bildung von elektronisch angeregten Zuständen oder Radikalen, insbesondere Sauerstoff-Radikalen, oder deren Folgeprodukte stimuliert. Dies geschieht bevorzugt durch Zugabe von Phorbol-Myristat-Azetat (PMA) in einer Endkonzentration von 20 - 30 ng/ml. Alternativ sind andere Dosierungen von PMA oder andere Stimuli einsetzbar (andere Phorbolester, Ionophore, Detergentien, Fluorid-Ionen, Stimulation über Fc- oder Komplementrezeptoren). Die bevorzugte PMA-Behandlung wird in einer Konzentration von 0,1 - 1000 ng/ml pro 1x10⁶ Zellen bei Temperaturen von 20 - 40 °C und Behandlungszeiten von 1 - 200 Minuten durchgeführt.

Typischerweise werden die Sauerstoffradikale in Form von Photonen (Luminometrie) sichtbar gemacht. Dies geschieht durch Zugabe eines chemilumigenen Substrats zu der Zellsuspension vor Initiierung der Radikalbildung. Üblicherweise wird Lucigenin in einer Konzentration von 1 mM verwandt; alternativ können andere Konzentrationen von Lucigenin eingesetzt werden. Ebenfalls möglich ist der Einsatz von Luminol oder Luminol-Derivaten (Isoluminol, ABEI, AHEI, ABEI-H; ABEI = N-(4-Aminobutyl)-N-ethyl isoluminol, ABEI-H = N-(4-Aminobutyl)-N-ethyl isoluminol hemisuccinamid, AHEI = N-(6-Aminohexyl)-N-ethyl isoluminol) oder Fluorescein und Derivaten als chemilumigenes Substrat. Die Messung nichtverstärkter Chemilumineszenz ist ebenfalls möglich. Weitere geeignete Verfahren zur Bestimmung der Radikalproduktion sind:

– Messung der $.O_2$-Produktion mittels spektrophotometrischen Nachweises der Reduktion von Cytochrom C [Cell. Immunol. 59 (1981) 301-318].

– Messung der $H_2O_2$-Produktion mittels Scopoletin, Phenolrot oder Oxidativ-Fluorogener Substrate (DCFH-DA, DCDCFH-DA: 2'7'-dichlorofluorescin diacetate, 5(and-6)-carboxy-2',7'-dichlorofluorescin diacetate). Bei Einsatz der letzten beiden ist auch zytofluorographische Messung möglich [J. Exp. Med. 156 (1982) 954-961]

– Licht-Mikroskopische Bestimmung des Prozentsatzes Radikalproduzierender Zellen mittels Nitroblue-Tetrazolium. [Blood 48 (1976) 308-313].

Die Bildung von elektronisch angeregten Zuständen oder Radikalen, insbesondere Sauerstoff-Radikalen und deren Folgeprodukte, kann ebenfalls erfaßt werden über Elektronen-Spinresonanz-Spektroskopie, Messung nativer Chemilumineszenz oder Energie-Transfer auf fluoreszente Substrate (Fluorimetrie) [Biochem. Biophys. Acta 192 (1969) 145-148].

In den genannten Meßsystemen bewirkt EPF eine deutliche Änderung der Bildung von elektronisch angeregten Zuständen oder Radikalen, insbesondere Sauerstoff-Radikalen und deren Folgeprodukte.

**Beispiel**

Nachweis von EPF in Seren von Frauen nach Embryotransfer

a) Modulation der Chemilumineszenz mononukleärer Zellen durch Frühschwangerschafts-Seren

Die erste Blutprobe wurde 1 - 10 Tage vor Embryotransfer entnommen; weitere Blutproben zwischen 5 und 11 Tage nach Embryotransfer. Der Erfolg des Transfers wurde durch die üblichen Zeichen der Schwangerschaft evaluiert (Ausbleiben der Regel, positiver HCG-Nachweis mit Radioimmunoassay).

Zu den pro Vial enthaltenen $1 \times 10^6$ mononukleären Zellen wurden 100 µl Serum zugegeben und für 30 Minuten bei 37 °C inkubiert. Danach wurden die Zellen in den Luminometer-Vials dreimal mit Hank's Balanced Salt Solution (HBSS) gewaschen. Um keine Zellen zu verlieren, wurde das Waschmedium nicht dekantiert, sondern bis auf ein definiertes Restvolumen von 500 µl abgesaugt, so daß die Serumkonzentration nach drei Waschungen unter 0,5 % lag.

Hiernach wurden pro Vial 10 µl einer Lucigenin-Lösung (12 mM in NaCl 0,9 %/Tris-HCL 0,01 m, pH 7,2) zugegeben und die Vials in die Meßkammern des Luminometers eingeführt. Nach Äquilibrierung der Temperatur auf 37 °C und Stabilisierung der Background-Chemilumineszenz wurden 12,5 µl Phorbol-Myristat-Azetat (1 µg/ml in MEM/BSA) als Stimulus zugegeben, entsprechend einer Endkonzentration von 25 ng/ml.

Als Maß der Sauerstoff-Radikal-Freisetzung durch die so behandelten Zellen wurde das Integral der Chemilumineszenz über 45 Minuten ab Zugabe des Phorbol-Esters gewählt. Da Seren verschiedener Patientinnen unabhängig von einer Schwangerschaft zu großen Unterschieden in der Sauerstoff-Radikal-Bildung der mononukleären Testzellen führten, wurden die Werte eines Patientinnen-"Testserums" jeweils in % des mit dem "Kontroll-Serum" dieser Patientin erhaltenen 45-Minuten-Integrals ausgedrückt. Der Wert des Kontrollserums wurde jeweils mit 100 % festgesetzt.

Der zur Hessung der Chemilumineszenz benutzte Biolumat LB 9595 (Berthold, Mildbad, BRD) erlaubt die parallele Wessung von 6 Proben. Dementsprechend wurden pro Meßreihe bis zu 6 Serumproben zusammengestellt. Um den Vergleich von Kontroll- und Test-Serum einer Patientin an möglichst vergleichbaren Zellon durchführen zu können, wurden grundsätzlich "Kontroll"- und "Testserum" einer Patientin im gleichen Testansatz untersucht.

b) Modulation der Chemilumineszenz einer humanen B-Lymphozyten-Linie ("HOLT") durch Frühschwangerschafts-Seren

Die "HOLT"-Zell-Linie, eine Epstein-Barr-transformierte B-Lymphozyten-Linie (J. Immunol. 133 (1984, 6) 3006) wurde in RPMI 1640 Medium mit 10 % fetalem Kälberserum in Kultur gehalten. Zum Chemilumineszenz-Test wurden Zellen aus der Kultur entnommen, zweimal mit MEM/BSA gewaschen und auf eine Dichte von $5 \times 10^6$/ml in MEM/BSA eingestellt. Pro Luminometer-Vial wurden 200 µl davon vorgelegt und bis zur weiteren Verwendung bei +4 °C aufbewahrt. Die weitere Durchführung des Chemilumineszenz-Tests war im wesentlichen identisch mit dem Vorgehen bei mononukleären Zellen. Die einzige Änderung bestand in der Verwendung höherer Dosen des Stimulus Phorbol-Myristat-Azetat (20-40 µg/ml).

Tabelle 1 zeigt die Beeinflussung der Chemilumineszenz mononukleärer Zellen (MNC) eines gesunden männlichen Spenders durch Patientinnenseren nach Embryotransfer aus der Klinik U.

Bei den Frauen, die schwanger wurden (C, E, I, J, L, F), liegen die Post-Transferwerte deutlich über 100 %. des Kontrollwertes.

Bei den Frauen, die nicht schwanger wurden (S, T, U), liegen die Post-Transferwerte etwa bei 100 % des Kontrollwertes.

Lediglich bei einer Frau (R) wird ein falsch positiver Wert gefunben.

Durch tägliche Behandlung mit HCG-Dosen von 2 - 10 Tausend I.E. werden die Unterschiede zwischen positiv und negativ verlaufenem Embryotransfer verwischt (Tabelle 2, Proben aus der Klinik H).

Tabelle 2 zeigt außerdem die unterschiedliche Beeinflussung der Chemilumineszenz von mononukleären Zellen (MNC) und B-Lymphozyten-Zell-Linien (BLCL). Bei erfolgreichem Embryotransfer (ET$^+$) wird bei der Verwendung mononukleärer Zellen eine Verstärkung und bei der Verwendung von B-Lymphozyten-Zell-Linien eine Verminderung der chemilumineszenz beobachtet.

## Tabelle 1

| Probe | Transfer am Zyklustag | Blutentnahme am Zyklustag | $\bar{x}$ | $\pm$ | SD | p< | (n) | Erfolg |
|---|---|---|---|---|---|---|---|---|
| U C | 14 | | | | | | | |
| C1 | | 10 | | | | | | |
| C2 | | 24 | 244 | ± | 151 | 0,05 | (7) | + |
| C3 | | 27 | 203 | ± | 34 | 0,1 | | |
| U E | 16 | | | | | | | |
| E1 | | 9 | | | | | | |
| E2 | | 24 | 126 | ± | 51 | NS | (7) | + |
| E3 | | 28 | 141 | ± | 37 | 0,1 | (4) | |
| U I | 17 | | | | | | | |
| I1 | | 9 | | | | | | |
| I2 | | 23 | 307 | ± | 193 | 0,1 | (5) | + |
| U J | 18 | | | | | | | |
| J1 | | 8 | | | | | | |
| J2 | | 23 | 181 | ± | 68 | 0,05 | (7) | + |
| U L | 16 | | | | | | | |
| L1 | | 7 | | | | | | |
| L2 | | 23 | 188 | ± | 98 | 0,1 | (6) | + |
| U F | 16 | | | | | | | |
| F1 | | 9 | | | | | | |
| F2 | | 24 | 340 | ± | 103 | 0,05 | (4) | + |

NS = Nicht Signifikant

Fortsetzung der

## Tabelle 1

| Probe | Transfer am Zyklustag | Blutentnahme am Zyklustag | Relative Chemilumineszenz $\bar{x}$ | $\pm$ | SD | p< | (n) | Erfolg |
|-------|-----------------------|---------------------------|-------------------------------------|-------|-----|-----|------|--------|
| U S   | 16                    |                           |                                     |       |     |     |      |        |
| S1    |                       | 8                         |                                     |       |     |     |      |        |
| S2    |                       | 24                        | 98                                  | $\pm$ | 80  | NS  | (3)  | –      |
| U T   | 18                    |                           |                                     |       |     |     |      |        |
| T1    |                       | 8                         |                                     |       |     |     |      |        |
| T2    |                       | 29                        | 120                                 | $\pm$ | 26  | NS  | (4)  | –      |
| U U   | 17                    |                           |                                     |       |     |     |      |        |
| U1    |                       | 8                         |                                     |       |     |     |      |        |
| U2    |                       | 25                        | 102                                 | $\pm$ | 35  | NS  | (3)  | –      |

NS = Nicht Signifikant

6

T a b e l l e   2

Zusammenfassung der Proben

| | KLINIK U | KLINIK H |
|---|---|---|
| MNC | $ET^+$ 215 $\pm$ 133 (n = 35) (p< 0,001) | $ET^+$ 96,5 $\pm$ 42 (n = 40) (NS) |
| | $ET^-$ 101 $\pm$ 28 (n = 20) (NS) | $ET^-$ 90,8 $\pm$ 48 (n = 32) (NS) |
| BLCL (HOLT) | $ET^+$ 67,1 $\pm$ 27 (n = 22) (p< 0,001) | $ET^+$ 117 $\pm$ 43 (n = 28) (NS) |
| | $ET^-$ 81,5 $\pm$ 14 (n = 8) (NS) | $ET^-$ 102 $\pm$ 26 (n = 20) (NS) |

$ET^+$ = Erfolgreicher Embryo-Transfer

$ET^-$ = Erfolgloser Embryo-Transfer

**Patentansprüche**

1. Verfahren zum frühen Nachweis einer Schwangerschaft durch Bestimmung von EPF (Early Pregnancy Factor) im Blut oder Harn oder in biochemischen Derivaten von Blut oder Harn von Schwangeren nach Ausschluß von Keimdrüsentumoren, dadurch gekennzeichnet, daß man die durch EPF beeinflußte Bildung von elektronisch angeregten Zuständen oder Radikalen, insbesondere Sauerstoff-Radikalen oder deren Folgeprodukte, einer Leukozytenpräparation oder menschlicher oder tierischer mononukleärer Zellen oder der "Holt"-B-Zell-Linie, der HL-60-Zell-Linie oder von Makrophagen-Zell-Linien mißt.

2. Verfahren nach Anspruch 1, dadurch gekennzeichnet, daß die menschlichen mononukleären Zellen Aveolar-Makrophagen, Peritonealzellen oder aus Knochenmark gewonnene Zellen sind.

3. Verfahren nach Anspruch 1, dadurch gekennzeichnet, daß die tierischen mononukleären Zellen Maus-Peritonealzellen oder aus Maus-Knochenmark gewonnene Zellen sind.

4. Verfahren zur Diagnostizierung von Keimdrüsentumoren nach Ausschluß einer Schwangerschaft, dadurch gekennzeichnet, daß man die durch EPF (Early Pregnancy Factor) beeinflußte Bildung von elektronisch angeregten Zuständen oder Radikalen, insbesondere Sauerstoff-Radikalen oder deren Folgeprodukte, einer Leukozytenpräparation oder menschlicher oder tierischer mononukleärer Zellen oder der "Holt"-B-Zell-Linie, der HL-60-Zell-Linie oder von Makrophagen-Zell-Linien mißt.

5. Verfahren nach Anspruch 1 zum Nachweis eines erfolgreichen Embryotransfers nach In-Vitro-Fertilisation.

6. Verfahren nach Anspruch 1 zum Nachweis einer Fertilisation nach einer künstlichen Insemination.

## Claims

1. Method for the early detection of pregnancy by determining EPF (Early Pregnancy Factor) in the blood or urine or in biochemical derivatives of blood or urine of pregnant females after excluding gonadal tumours, characterised in that there is measured the EPF-influenced generation of electronically excited states or radicals, especially oxygen radicals or their secondary products, which generation is brought about by a leucocyte preparation or by human or animal mononuclear cells or by the "Holt"-B cell line, the HL-60 cell line or macrophage cell lines.

2. Method according to claim 1, characterised in that the human mononuclear cells are alveolar macrophages, peritoneal cells, or cells obtained from bone marrow.

3. Method according to claim 1, characterised in that the animal mononuclear cells are murine peritoneal cells, or are cells obtained from murine bone marrow.

4. Method for diagnosing gonadal tumours after excluding pregnancy, characterised in that there is measured the generation, influenced by EPF (Early Pregnancy Factor), of electronically excited states or radicals, especially oxygen radicals or their secondary products, which generation is brought about by a leucocyte preparation or by human or animal mononuclear cells or by the "Holt"-B cell line, the HL-60 cell line or macrophage cell lines.

5. Method according to claim 1 for detecting successful embryo transfer after in vitro fertilisation.

6. Method according to claim 1 for detecting fertilisation after artificial insemination.

## Revendications

1. Procédé pour déceler précocement une grossesse par un dosage d'EPF (Early Pregnancy Factor) dans le sang ou l'urine ou dans des dérivés biochimiques de sang ou d'urine de femmes enceintes, après avoir exclu la présence de tumeurs ovariennes, procédé caractérisé en ce que l'on mesure la formation, influencée par l'EPF, de radicaux ou états activés par électrons, en particulier de radicaux d'oxygène ou de dérivés de ceux-ci, d'une préparation leucocytaire ou de cellules mononucléaires humaines ou animales, ou de la lignée cellulaire "Holt"-B, de la lignée cellulaire HL-60 ou de lignées de macrophages.

2. Procédé selon la revendication 1, caractérisé en ce que les cellules mononucléaires humaines sont des macrophages alvéolaires, des cellules péritonéales ou des cellules de moelle osseuse.

3. Procédé selon la revendication 1, caractérisé en ce que les cellules mononucléaires animales sont des cellules péritonéales de souris ou des cellules de moelle osseuse de souris.

4. Procédé pour diagnostiquer la présence de tumeurs ovariennes alors qu'une grossesse a été exclue, procédé caractérisé en ce que l'on mesure la formation, influencée par l'EPF (Early Pregnancy Factor), de radicaux ou d'états activés par électrons, en particulier de radicaux d'oxygène ou de leurs dérivés, d'une préparation de leucocytes ou de cellules mononucléaires humaines ou animales, ou bien de la lignée cellulaire B "Holt", de la lignée cellulaire HL-60 ou de lignées de macrophages.

5. Procédé selon la revendication 1 pour mettre en évidence la réussite d'un transfert d'embryon après une fécondation in vitro.

6. Procédé selon la revendication 1 pour mettre en évidence une fécondation après une insémination artificielle.